(19) Europäisches Patentamt
European Patent Office
Office européen des brevets

(11) **EP 2 699 970 B1**

(12) **EUROPÄISCHE PATENTSCHRIFT**

(45) Veröffentlichungstag und Bekanntmachung des Hinweises auf die Patenterteilung:
**28.11.2018 Patentblatt 2018/48**

(21) Anmeldenummer: **12717602.2**

(22) Anmeldetag: **16.04.2012**

(51) Int Cl.:
*G03H 1/00* *(2006.01)*  *G03H 1/04* *(2006.01)*
*A61B 5/00* *(2006.01)*  *G01B 9/02* *(2006.01)*
*G01N 21/47* *(2006.01)*

(86) Internationale Anmeldenummer:
**PCT/EP2012/001639**

(87) Internationale Veröffentlichungsnummer:
**WO 2012/143113 (26.10.2012 Gazette 2012/43)**

(54) **VERFAHREN ZUR OPTISCHEN TOMOGRAPHIE**

METHOD FOR OPTICAL TOMOGRAPHY

PROCÉDÉ DE TOMOGRAPHIE OPTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.04.2011 DE 102011018603**
**18.07.2011 US 201161508831 P**

(43) Veröffentlichungstag der Anmeldung:
**26.02.2014 Patentblatt 2014/09**

(73) Patentinhaber:
 • **Universität zu Lübeck**
**23562 Lübeck (DE)**
 • **Medizinisches Laserzentrum Lübeck GmbH**
**23562 Lübeck (DE)**
 • **Thorlabs GmbH**
**85221 Dachau (DE)**

(72) Erfinder:
 • **HILLMANN, Dierck**
**23562 Lübeck (DE)**
 • **HÜTTMANN, Gereon**
**23564 Lübeck (DE)**
 • **KOCH, Peter**
**23558 Lübeck (DE)**
 • **LÜHRS, Christian**
**23881 Niendorf (DE)**
 • **VOGEL, Alfred**
**23568 Lübeck (DE)**

(74) Vertreter: **Hermann, Felix**
**Boehmert & Boehmert**
**Anwaltspartnerschaft mbB**
**Pettenkoferstrasse 22**
**80336 München (DE)**

(56) Entgegenhaltungen:
 • **POTCOAVA M C ET AL: "Optical tomography for biomedical applications by digital interference holography", MEASUREMENT SCIENCE AND TECHNOLOGY, IOP, BRISTOL, GB, Bd. 19, Nr. 7, 1. Juli 2008 (2008-07-01), Seiten 74010/1-74010/8, XP020136304, ISSN: 0957-0233, DOI: 10.1088/0957-0233/19/7/074010 [gefunden am 2008-05-23]**

EP 2 699 970 B1

**Beschreibung**

Die Erfindung betrifft ein Verfahren zur Erfassung räumlich strukturierter Probenvolumina mittels kohärentem Licht und Digitaler Holographie (DH). Die Erfindung betrifft ebenso ein Verfahren zur Untersuchung der Tiefenstruktur von Proben nach Art der Optischen Kohärenztomographie ("optical coherence tomography", OCT).

Holographie umfasst die dreidimensionale Ablichtung von Objekten mit kohärentem Licht, insbesondere mit Laserlicht. Das Licht wird dabei durch einen Strahlteiler in Proben- und Referenzarm aufgeteilt, wobei der Probenstrahl die Probe beleuchtet. Von der Probe in verschiedenen Abständen reflektiertes bzw. zurück gestreutes Licht wird auf eine lichtempfindliche Fläche (z. B. den Film in der Kamera) geführt und dort mit dem Referenzlicht überlagert. In der Bildebene entsteht durch Interferenz eine räumliche Lichtintensitätsverteilung, das Hologramm.

Das Hologramm wird bei der klassischen Holographie optisch rekonstruiert, indem man das entwickelte Hologramm erneut mit einem Referenzstrahl beleuchtet. Das dabei entstehende Beugungswellenfeld besitzt eine Komponente, die dem ursprünglichen Objektwellenfeld entspricht. Weitere Komponenten repräsentieren ein konjugiertes Bild, sowie die Referenzwelle, deren Anteil proportional zur ursprünglichen Intensität von Referenz und Objektwelle ist.

In der Digitalen Holographie wird die lichtempfindliche Fläche durch einen elektronischen Lichtsensor (z. B. CCD- oder CMOS-Kamera) gebildet. Ein digital gespeichertes Hologramm kann ohne den Umweg einer optischen Rekonstruktion der weiteren Auswertung in einer Recheneinheit zugeführt werden. Beispielsweise kann in Kenntnis des Lichtwellenfeldes des Referenzstrahls das Lichtwellenfeld des von der Probe wiederkehrenden Lichts in beliebigen Ebenen parallel zur Kameraebene berechnet werden. Wenn man das Lichtwellenfeld in der Kameraebene kennt, kann man es numerisch propagieren lassen, etwa durch Anwenden der "Angular Spectrum" Methode, der Fresnel-Transformation oder einer anderen Integraltransformation.

Der in vielen Fällen angewandte "Angular Spectrum"-Ansatz bzw. "Parallel Wave Expansion"-Ansatz zur Berechnung des Wellenfeldes in einer Rekonstruktionsebene beruht auf einer Entwicklung des Objektwellenfeldes in der Hologrammebene $f(x, y, z = 0)$ in ebene Wellen $\exp(i(k_x x + k_y y))$, d. h. es wird die 2D-Fouriertransformierte des Wellenfeldes berechnet. Damit sind die transversalen Anteile der Wellenzahlen der einzelnen ebenen Wellen des Wellenfeldes bekannt, also die Funktion $\tilde{f}(k_x, k_y, z = 0)$. Der Begriff "transversal" soll im Folgenden stets auf Richtungen in der Hologrammebene verweisen. Die Richtung senkrecht zur Hologrammebene wird durch das Adjektiv "axial" notiert. Die Hologrammebene wiederum fällt apparativ mit der Ebene zusammen, in der eine Messung von Lichtintensitäten erfolgt (-> Kameraebene, s. u.).

Ein Propagieren des Feldes entspricht nun dem Anpassen der Phasen dieser Komponenten, wie sie in einer vorgegebenen Distanz zur ursprünglichen Ebene sein müssen. Dies geschieht durch Multiplikation jeder einzelnen Komponente $\tilde{f}(k_x, k_y, z = 0)$ mit dem Phasenfaktor $\exp(i k_z z_0)$, wobei $k_z$ die axiale Komponente des Wellenzahlvektors bezeichnet und für alle Komponenten $(k_x, k_y)$ i. a. verschieden ist. Unter $z_0$ ist die axiale Propagationsdistanz zu verstehen. Die Komponente $k_z$ ist (im Vakuum) gegeben als

$$k_z(k, k_x, k_y) = \sqrt{k^2 - k_x^2 - k_y^2}, \qquad (1)$$

wobei $k = 2\pi/\lambda$ die Gesamtwellenzahl darstellt. Mathematisch ausgedrückt berechnet man folglich die propagierte Ebenen-Wellen-Zerlegung aus der ursprünglichen (i. a. die Zerlegung des gemessenen Wellenfeldes) mittels

$$(2) \qquad \tilde{f}(k_x, k_y z = z_0) = \exp\left(i z_0 \sqrt{k^2 - k_x^2 - k_y^2}\right) \tilde{f}(k_x, k_y, z = 0).$$

So ist es möglich, die Wellenfeldverteilung des gestreuten Lichtes in beliebigen Ebenen außerhalb und innerhalb der Probe zu berechnen. Es kann insbesondere nach der Aufzeichnung des elektronischen Hologramms eine Rekonstruktion des Lichtwellenfeldes in irgendeiner wählbaren "Rekonstruktionsebene" erfolgen.

Bei Volumina mit hintereinanderliegenden teiltransparenten Strukturen (z. B. biologisches Gewebe) setzt sich das Wellenfeld aus Anteilen zusammen, die in verschiedenen Abständen zur Kameraebene reflektiert worden sind bzw. von Streuern in verschiedenen Tiefenebenen (parallel zur Hologrammebene) gestreut wurden. Bei der Rekonstruktion eines reellen Bildes des Volumens erscheint das Wellenfeld in der Rekonstruktionsebene scharf, während alle anderen Tiefenebenen des Volumens einen unscharfen Anteil zum rekonstruierten Bild beitragen.

Digitale Holographie eignet sich sehr gut zur schnellen Erfassung der Oberfläche einer Probe, wenn diese in mehr als nur einer definierten Fokusebene liegt (etwa, weil die Oberfläche gekrümmt ist). Weist die Probe aber Streustrukturen in verschiedenen Tiefen auf, die axial hintereinander liegen, dann interferieren auch die Wellenfeldanteile solcher Streuer miteinander und verfälschen das Bild.

Es wurde bereits erkannt, dass sich mit Hilfe der Digitalen Holografie tomographische Daten rekonstruieren lassen, wenn man mehrere Wellenlängen (z. B. durchstimmbarer Laser) verwendet und für eine - vorzugsweise äquidistant gewählte - Folge von Wellenzahlen je ein Hologramm aufzeichnet. Die aufgezeichneten Hologramme werden alle für dieselbe

Rekonstruktionsebene rekonstruiert, und das dabei entstehende komplexe Wellenfeld wird überlagert. Bei dieser numerischen Superposition der Wellen mit unterschiedlichen

Wellenlängen interferieren nur jene Anteile, die aus der Fokusebene stammen, alle anderen Anteile werden unterdrückt. Das Verfahren wiederholt man dann für weitere Ebenen, um sukzessive ein Tiefenbild der Probe in der Zusammenschau aller Ebenen zu gewinnen. Eine solche Vorgehensweise ist numerisch sehr aufwendig und für die Tomographie größerer Volumina nur schwer praktikabel. (Wavelength-Scanning Digital Interference Holography: L. Yu, M.K. Kim, Variable tomographic scanning with wavelength scanning digital interference holography, Optics Communications 260 (2006) 462-468; M.K. Kim, Principles and techniques of digital holographic Microscopy, SPIE Reviews 018005-1 Vol. 1, 2010 und Potcoava et al., "Optical tomography for biomedical applications by digital interference holography",Measurement Science and Technology, IOP, Bristol, GB, BRISTOL, GB, Bd. 19, Nr. 7, 1. Juli 2008 (2008-07-01), Seiten 7 4010/1-7 4010/8,).

In der Arbeit von Blazkiewicz et al. Experimental Demonstration of Signal-to-Noise-Ratio Improvement of Fourier-Domain Optical Coherence Tomography (1. P. Blazkiewicz, M. Gourlay, J. R. Tucker, A. D. Rakic, and A. V. Zvyagin, "Signal-to-noise ratio study of full-field fourier-domain optical coherence tomography," Appl. Opt. 44, 7722-7729 (2005).) wird vorgeschlagen, ein Fourier-Domain (FD-)OCT-System mit Fourier-Holographie zu kombinieren, wobei die Tiefeninformation parallel auf allen Kamerapixeln für alle Messpunkte der Probe erfasst wird. Ziel der Arbeit ist, das Abrastern der Probe mit dem OCT-Messstrahl zu vermeiden. Die Probe wird dabei flächig beleuchtet und das gestreute Licht wird mit einer Linse ins Unendliche abgebildet und auf die Kamera gebracht, d. h. die Kamera wird im Beugungsfernfeld der Objektstrukturen angeordnet. Für mehrere Wellenlängen einer durchstimmbaren Laserlichtquelle wird ein Fourier-Hologramm erzeugt, und die Gesamtheit aller Hologramme stellt praktisch die 3D-Fouriertransformierte der Probenstruktur dar. Allerdings ist der optische Aufbau nur in der Lage, die Tiefenumgebung einer gewählten Fokusebene in der Probe ins Fernfeld abzubilden, und damit ergibt sich auch nur für diese Ebene eine scharfe Rekonstruktion. Dieses System ist daher nicht in der Lage, alle Ebenen der Probe scharf zu rekonstruieren, ohne dass hierfür eine Neuausrichtung der Messapparatur erforderlich wäre. Mit Erhöhung der transversalen Auflösung wird der Bereich der Probe, der scharf abgebildet werden kann, drastisch kleiner, da bei abbildenden Systemen einen fundamentalen Zusammenhang zwischen Schärfentiefe und transversaler Auflösung existiert. Damit steigt die Zahl der notwendigen Messungen mit verschobener Fokuslage sogar quadratisch mit der gewünschten transversalen Auflösung.

Die Patentschrift US 7,602,501 B2 befasst sich ausführlich mit der Auswertung wellenlängenabhängiger, zweidimensionaler Interferogramme zur Berechnung des dreidimensionalen Streuvermögens einer Probe, wobei auf bewegte Teile im Messaufbau (z.B. für Phasenmodulation oder Refokussierung) verzichtet werden kann. Die Druckschrift lehrt, die Messdaten als Ergebnis eines Modells der Lichtstreuung in der Probe zu formulieren und dieses Modell sodann zu invertieren, um ein dreidimensionales Streumodell der Probe zu erhalten. Die Invertierung eines a priori nicht eindeutig invertierbaren Integralkerns, stellt ein "ill-posed problem" dar, welches im Allgemeinen nur mit Regularisierungsmaßnahmen gelöst werden kann. Auch die US 7,602,501 B2 verwendet eine Fokusebene in der Probe, die auf eine elektronische Kamera abgebildet wird, wie dies in der OCT üblich ist. Sie erzielt durch die Auswertung höhere Auflösung der Streustrukturen auch deutlich außerhalb dieser Fokusebene, so dass ein Verschieben der Fokusebene zum Scannen des gesamten Probenvolumens nicht mehr nötig ist.

In der US 7,602,501 B2 wird nicht erkannt, dass es günstiger sein kann, auf eine abbildende Optik vollständig zu verzichten. Dieser Verzicht gestattet insbesondere die Anwendung von Methoden der Digitalen Holographie, mit denen es vermeidbar wird, die Berechnung der Probenstreustärke als Invertierung eines Streumodells auszuführen. Vielmehr kann man dann eine - numerisch simulierte - Messung der Streustärken in beliebigen Probentiefen vornehmen.

Der Konferenzbeitrag von Shabanov et al., "Broadband 3-D Digital Holography for Depth Structure Visualization" zur Konferenz Digital Holography and Three-Dimensional Imaging (DH), Miami, USA, April 2010, präsentieren ein Verfahren zur 3D-Visualisierung der Tiefenstruktur streuender biologischer Proben mit hoher räumlicher Auflösung (10-15 μm) ohne Abtastung in longitudinaler oder transversaler Richtung. Dieses Verfahren basiert auf einer holographischen, digitalen Aufzeichnung von am Objekt gestreuter Lichtwellen für eine diskrete Menge von Spektralkomponenten und einer digitalen Wiederherstellung des 3D-Bild a-posteriori.

Der Messaufbau von Shabanov et al. verwendet neben einer Digitalkamera und einer durchstimmbaren Laserlichtquelle auch einen Phasenmodulator zur Variation der Referenzarmlänge. Für eine Anzahl N benutzter Laserlichtwellenlängen werden jeweils drei Bilder mit verschiedenen Referenzlichtphasen aufgezeichnet, d.h. es werden 3 * N Bilder zur nachträglichen Auswertung erfasst und gespeichert.

Die Aufgabe der Erfindung ist es, ein Verfahren zur 3D-Bildgebung streuender Proben vorzuschlagen, bei dem eine gleichmäßig hohe dreidimensionale Auflösung auch über einen großen Tiefenbereich der Probe bei zugleich minimaler Belastung der Probe durch die Bestrahlung erreicht wird.

Die Aufgabe wird gelöst durch ein Verfahren nach Anspruch 1. Die Unteransprüche geben vorteilhafte Ausgestaltungen an.

Das Ziel der Erfindung ist die Ermittlung des dreisimensionalen Streuvermögens S(x,y,z) der Probe. Ist dieses numerisch - in einer akzeptablen Auflösung, d.h. Stützstellendichte - bekannt, dann kann mit an sich bekannten Methoden jede beliebige Darstellung dreidimensionaler Datenarrays zur Bildgebung herangezogen werden, z.B. die Berechnung beliebiger zweidimensionaler Schnitte. Nachfolgend wird beschrieben, wie sich S(x,y,z) für die gesamte Probe - gemeint ist hier der Probenbereich, für den das aus der Probe zurück gestreute Licht überhaupt auf den Detektor gelangen kann - mit nur einer Messung und mit nur einem numerischen Auswerteschritt bestimmen läßt.

Die Erfindung kombiniert die Digitale Holographie mit der Fourier Domain (FD-)OCT in neuartiger Weise. Zuerst werden wellenlängenabhängige Hologramme der Probe mit einer elektronischen Kamera aufgezeichnet. Das Referenzlicht wird in an sich bekannter Weise dazu benutzt, die elektrischen Wellenfelder des in der Probe gestreuten Lichts in der Kameraebene zu berechnen und in einer axial verschobenen Rekonstruktionsebene zu rekonstruieren. Diese Rekonstruktionsebene kann insbesondere im Innern der Probe liegen. Der Erfindungsgedanke besteht darin, das rekonstruierte Wellenfeld in der Rekonstruktionsebene erneut mit einem Referenzlichtstrahl - im Rechner - zu überlagern, um ein zweidimensionales, nach Art der OCT auswertbares, numerisches Interferogramm zu erhalten. Die pixelweise OCT-Auswertung des Interferogramms liefert Tiefeninformation aus einer Schicht der Probe in der Umgebung der Rekonstruktionsebene. Gewissermaßen wird das Verschieben der OCT-Kamera in eine beliebige Ebene, insbesondere ins Probeninnere, numerisch simuliert.

Da die Rekonstruktionsebene frei wählbar ist, kann mit der Erfindung jede Probentiefe analysiert werden, und es ergibt sich ein dreidimensionales Bild des gesamten Probenvolumens. Die erforderlichen Messdaten werden in einer Messung vor der numerischen Analyse aufgezeichnet und stellen nur eine minimale Belastung der Probe durch Licht dar.

Besonders vorteilhaft an der Erfindung ist, dass die numerische Auswertung tatsächlich nicht Schicht für Schicht erfolgen muss, sondern vielmehr die Berechnung der dreidimensionalen Streustärke S(x,y,z) effektiv in einem Rechenschritt erfolgen kann, der eine Fouriertransformation auf nicht-äquidistanten Stützstellen umfaßt.

Die Erfindung wird nachfolgend näher erläutert:
Die Probe wird mit einer durchstimmbaren Laserlichtquelle beleuchtet. Ein möglichst großer Anteil des von der Probe zurück gestreuten Lichtes soll von einer elektronischen Kamera, einem 2D-Lichtdetektor-Array, erfasst werden können. Deshalb wird die Kamera möglichst nahe an der Probe angeordnet, so dass die Krümmung der Lichtwellenfronten nicht mehr vernachlässigt werden kann. Es wird erfindungsgemäß also das "Fresnel-Beugungsbild" oder auch das "Beugungsnahfeld" der Probe aufgezeichnet. Die Kamera soll dabei Licht erfassen können, das maximal unter einem Winkel $\alpha_{max}$ gegenüber der optischen Achse austritt. $\alpha_{max}$ bestimmt die numerische Apertur NA = $n \cdot \sin \alpha_{max}$ der Anordnung, wobei $n$ der Brechungsindex des Mediums ist.

Aus der Laserlichtquelle wird durch Strahlteilung ein Referenzlichtstrahl entnommen und auf die Kameraebene geführt. Die Beleuchtung mit Referenzlicht erfolgt im Allgemeinen derart, dass eine vorbestimmte Anzahl von Kamerapixeln vom Referenzlicht möglichst gleichmäßig beleuchtet wird.

Es sei darauf hingewiesen, dass die Kameraebene ebenso durch eine Ebene ersetzt werden kann, in der ein optisches Abbild des 2D-Lichtdetektor-Arrays vorliegt. Die abbildende Optik führt dann das in der Ebene des Abbildes eintreffende Licht weiter auf den physikalischen Detektor. Der Einfachheit halber wird im Folgenden von der Kameraebene in dem Sinne die Rede sein, dass dort per Definition eine physikalische Messung von Lichtintensitäten erfolgt, gleichgültig, ob sich das 2D-Lichtdetektor-Array oder nur sein Abbild in eben dieser Kameraebene befindet.

Beim erfindungsgemäßen Verfahren wird die Probe flächig beleuchtet. Das aus der Probe zurück gestreute Licht wird direkt auf die Kameraebene geführt. Es existiert kein Fokus des Probenlichtstrahls in der Probe, wie dies etwa beim konfokalen OCT ansonsten üblich ist. Insbesondere wird keine Ebene der Probe durch das Probenlicht scharf auf die Kameraebene abgebildet.

Für die Detektion von Hologrammen in der Kameraebene ist der Winkel zwischen Proben- und Referenzlichtstrahlen wichtig. Der Winkel zwischen den lokalen Tangenten auf der Referenz- und Probenwellenfront bestimmt die lokale räumliche Modulationsfrequenz des Hologramms. Treffen Referenz- und Probenlicht der Wellenlänge λ unter einem Winkel $\theta$ auf den Sensor, so ist die lokale räumliche Frequenz gegeben durch $f \approx \frac{2}{\lambda} \sin \frac{\theta}{2}$. Diese Modulationsfrequenz muss von der Kamera ohne Artefakte abgetastet werden können. Daher begrenzen das Auflösungsvermögen und die Pixelzahl digitaler Kameras den maximalen Winkel zwischen Proben- und Referenzstrahlung und damit auch das Bildfeld und die verwendbare NA.

Um auf möglichst vielen Pixeln des 2D-Lichtdetektor-Arrays möglichst kleine Winkel zwischen Referenz- und Probenwellenfront zu gewährleisten, wird der durch Lichtstreuung den Probenstrahl aufweitende Effekt der Probe vorzugsweise durch eine Aufweitung des Referenzstrahls nachvollzogen. Es bietet sich an, ein strahlaufweitendes Element z. B. einen sphärischen Spiegel im Referenzarm anzuordnen. Der Referenzstrahl wird vorzugsweise sphärisch aufgeweitet, so dass auf dem Detektor kugelförmige Wellenfronten auftreffen. In diesem Sinne ist der Referenzstrahl somit divergent und besitzt einen - virtuellen - Fokus im Bereich der Probe, d. h. in einem Abstand zur Kameraebene, der dem Abstand der Probe zur Kameraebene etwa entspricht. Dieser virtuelle Fokus des Referenzlichts soll als Referenzfokus bezeichnet werden.

Die geringsten lokalen Winkel - also die geringsten Modulationsfrequenzen im Hologramm - entstünden, wenn der Referenzfokus mittig im Messvolumen läge. Dies wäre allerdings für die Rekonstruktion der Lichtwellenfelder ungünstig, da man dann ein Fourier-Hologramm erhielte und keine scharfe Abbildung aller Ebenen eines Volumens erreichen würde, wenn das Volumen deutlich tiefer als die Rayleigh-Länge $l_R = \lambda/\pi NA^2$ ist.

Fourier-Hologramme lassen sich zudem im Allgemeinen in der praktischen Messung nicht vollständig von ihrem konstanten Hintergrund (DC-Anteil) befreien. Bleibt nach dem Umrechnen der gemessenen Hologramme in elektrische Feldstärken der DC-Anteil erhalten, so ist dieser nach der Multiplikation mit der (konjugierten) Referenzwelle proportional zur Referenzwelle - einer Punktquelle, die ihren Ursprung im Messvolumen hat. Propagiert man das Feld in das Messvolumen an den Ort des Referenzfokus, so rekonstruiert man vor allem diese Punktquelle, was dann zu einem dominanten hellen Spot in den Daten führt, der das rekonstruierte Volumen überdeckt.

Vorzugsweise wird daher der Referenzfokus so festgelegt, dass er möglichst nahe am Messvolumen, jedoch außerhalb des Messvolumens liegt. Bei bekanntem Abstand des abzubildenden Probenvolumens zur Kameraebene ist das den Referenzstrahl aufweitende Element angemessen einzurichten, beispielsweise durch Vorgabe von Abstand und/oder Krümmungsradius des sphärischen Spiegels.

Die Kamera zeichnet während des Durchstimmens der Laserlichtquelle, welches vorzugsweise zwischen zwei Intervallgrenzen der Wellenlängen monoton auf- oder absteigend erfolgt, eine Mehrzahl von Hologrammen auf. Es werden $N$ Hologramme für $N$ bekannte Wellenlängen aufgezeichnet. Dabei ist $N > 100$ und mindestens doppelt so groß wie die Zahl der Tiefenstützstellen in der Probe, zu denen man Streustärken bestimmen möchte. Besonders bevorzugt ist sogar $N > 1000$. Mit heute verfügbaren Laserquellen und Hochgeschwindigkeitskameras kann bei einer Auflösung von 1024 × 1024 Pixeln und einer Framerate von 500 fps im derzeit existenten Messaufbau die vollständige Messung einer Probe in 2,1 Sekunden erfolgen.

Eine kürzere Messzeit, vorzugsweise unter 1 Sekunde, besonders bevorzugt unter 200 ms, ist günstig, wenn von einer Eigenbewegung der Probe (z.B. lebendes Gewebe) ausgegangen werden muss, weil Dopplerverschiebungen des Laserlichts das Verfahren besonders störanfällig gegenüber Bewegungen macht. Dies ist bei medizinischen Anwendungen, etwa bei der Untersuchung des Auges, der Fall. Schränkt man die Anforderungen hinsichtlich Auflösung und/oder Probenvolumen ein, lassen sich solche Messzeiten bereits heute erreichen.

Prinzipiell landet jedes im Winkelbereich der NA aus der Probe zurück gestreute Photon auf der Flächenkamera und kann bezüglich seiner Laufrichtung und Lauflänge in der Probe ausgewertet werden. Für ballistische (einfach gestreute) Photonen kann so der exakte Ursprung der Streuung bestimmt werden.

In Analogie zur Digitalen Holographie werden die erfassten Hologramme in Kenntnis der jeweils vorliegenden Referenzwelle für jede Wellenlängen $\lambda_n$ in komplexe Lichtwellenfelder $E_n(x, y, k_n = {}^{2\pi}/\lambda_n)$, $n = 1,..., N$ mit $x$ und $y$ als Pixelkoordinaten der Kamera umgerechnet, wobei zusätzliche Terme, die aus dem DC-Anteil und der konjugierten Welle entstehen, zunächst vernachlässigbar sind. Konkret wird die in der Kameraebene detektierte, auf den Pixeln registrierte Lichtintensität - das Hologramm - durch die vorbekannte Referenzwelle dividiert. Das so bestimmte Wellenfeld des gestreuten Probenlichts läßt sich dann in an sich bekannter Weise axial numerisch propagieren (hier mit der Angular Spectrum Methode wie, dargelegt im Stand der Technik s. o.).

Die Grundidee der Erfindung ist nun, die Laufzeitverteilung der propagierten Wellenfelder in der Rekonstruktionsebene in Bezug auf das Referenzlicht für alle $k_n$ auszuwerten, um zusätzliche Tiefeninformation über die Umgebung der Rekonstruktionsebene zu erhalten. Man kann dies als eine Art simulierte OCT-Messung in der Rekonstruktionsebene - ggf. im Innern der Probe - ansehen, wobei hier aber keine physikalischen Überlagerung oder Intensitätsdetektion mehr stattfindet. Vielmehr erfolgen alle weiteren Schritte ausschließlich im Rechner.

Die Laufzeiten sind bereits im Prinzip in den berechneten Phasendaten vorhanden, werden jedoch durch die zuvor ausgeführten Rechenschritte sozusagen "verwischt". Ihre Wiederherstellung gelingt jedoch ohne Weiteres durch eine Anpassung der Phasen aller Raumfrequenzspektren $E_n(k_x, k_y, k_n)$ der propagierten Lichtwellenfelder. Als transversale Raumfrequenzspektren werden im Folgenden die zweidimensionalen Fouriertransformierten der Lichtwellenfelder entlang der Achsen, die die Rekonstruktionsebene bzw. Kameraebene aufspannen, bezeichnet.

Bei der ursprünglichen Messung der Hologramme repräsentieren die Phasendifferenzen korrekt die Laufzeitunterschiede zwischen Referenz- und Probenlicht. Wird aus den gemessenen Intensitäten nun durch Multiplikation mit der konjugierten Referenzwelle das elektrische Wellenfeld in der Kameraebene berechnet, so spiegeln die erhaltenen Phasendifferenzen die Laufzeitunterschiede zwischen Probe und Kamera wider, vorausgesetzt, das Referenzwellenfeld wird phasenkorrekt berechnet, d.h. insbesondere mit einer über alle Wellenzahlen konstanten Phase in der Referenzebene. Um eine phasenneutrale Multiplikation mit der Referenzwelle zu gewährleisten, muss man mit einer zusätzlichen Phase multiplizieren, die die eingeführte Laufzeit kompensiert.

Für eine ebene Welle benutzt man daher beispielsweise einen konstanten Phasenfaktor statt einer ebenen Welle mit einer Laufzeit entsprechend ihrer zurückgelegten Distanz z, d.h. exp(ikz) wird durch exp(iφ) ersetzt, wobei φ

eine beliebige von k unabhängige Konstante darstellt.

Ein weiteres Beispiel ist eine sphärische Welle R(x,y;k) mit beliebiger Laufzeit in der Kameraebene.

$$(3) \qquad R(x,y;k) = \exp\left(ik\sqrt{R^2 + x^2 + y^2}\right)$$

Man kompensiert diese durch Einfügen eines zusätzlichen Phasenfaktors, so dass R(x,y;k) für ein beliebiges Koordinatenpaar (x,y) nicht mehr von der Wellenzahl k abhängt, d. h. man ersetzt beispielsweise R(x,y;k) durch R'(x,y;k)

$$(4) \qquad R'(x,y;k) = \exp\left(ik\sqrt{R^2 + x^2 + y^2} - ikR\right)$$

Für das Koordinatenpaar (x,y)=(0,0) ist R' dann unabhängig von der Wellenzahl k.

Bei der Propagation einer Welle muss eine ähnliche Kompensation vorgenommen werden. Die Propagation des transversalen Raumfrequenzspektrums $f(k_x,k_y,z=0;k)$ in eine andere E-bene geschieht mittels

$$(5) \qquad f(k_x, k_y, z; k) = \exp\left(iz\sqrt{k^2 - k_x^2 - k_y^2}\right) f(k_x, k_y, 0; k)$$

Um bei diesem Schritt keine Laufzeitverschiebung einzuführen, muss der Propagationskern ebenfalls für irgendeine feste transversale Raumfrequenz $(k_x,k_y)$ unabhängig von der Wellenzahl k sein. Man passt den Propagator beispielsweise so an:

$$(6) \qquad f(k_x, k_y, z; k) = \exp\left(iz\sqrt{k^2 - k_x^2 - k_y^2} - izk\right) f(k_x, k_y, 0; k),$$

Bei dieser bevorzugten Wahl der Phasenanpassung erfahren die Komponenten der transversalen Raumfrequenzspektren zu $(k_x,k_y)=(0,0)$ keine von k abhängige Phasenverschiebung.

Als eine allgemeine Regel wird jedes transversale Raumfrequenzspektrum der Lichtwellenfelder nach Propagation in die Rekonstruktionsebene mit einem Phasenfaktor abhängig von $k_n$ multipliziert, so dass die Komponenten $E_n(k_{x0},k_{y0},k_n)$ aller N Raumfrequenzspektren in der Rekonstruktionsebene für eine vorbestimmte transversale Raumfrequenz $(k_{x0}, k_{y0})$ denselben Wert annehmen. Die Wahl von $k_{x0}$ und $k_{y0}$ ist an sich beliebig, bevorzugt wird man aber $k_{x0} = k_{y0} = 0$ wählen, da der erforderliche Phasenfaktor dann die einfachste Gestalt hat.

Aus den gemessenen Hologrammen kann hiernach eine dreidimensionale Streustärkenverteilung in der Umgebung der Rekonstruktionsebene berechnet werden nach der Formel

$$(7) \qquad S(x,y,z) = \mathcal{F}_{2k \to z}^{-1}\left[ \mathcal{F}_{2D}^{-1} e^{-ikz_0} e^{ik_z(k,k_x,k_y)z_0} \mathcal{F}_{2D}\left[E(x,y;k)\right]\right],$$

wobei $E(x, y, k)$ das berechnete Wellenfeld in der Kameraebene, $z_0$ die gewünschte Rekonstruktionsebene, $F_{2D}$ die zweidimensionale Fouriertransformation entlang $x$ und $y$ und $F$ die eindimensionale Fouriertransformation von k nach z bezeichnet.

Das gemäß (7) rekonstruierte Volumen wird aber nur in der Tiefenumgebung um $z = z_0$ in transversaler Richtung scharf abgebildet. Die Streustärke der Probe wird somit zunächst nur für eine relativ dünne Schicht der Probe bei $z_0$ so genau bestimmt, dass man von einer Bildgebung der Probe sprechen kann. Die Dicke der besagten Schicht ist näherungsweise durch die Rayleigh-Länge, d. h. durch die numerische Apertur (NA) des Messaufbaus, gegeben. Mit steigender NA nimmt die Rayleigh-Länge jedoch ab. Andererseits gilt in Analogie zur Digitalen Holographie: Je größer die NA ist, desto höher ist die transversale Auflösung.

Demgegenüber ist die axiale Auflösung - innerhalb der Begrenzung der Schichtdicke durch die Rayleigh-Länge - wie bei der FD-OCT durch die Bandbreite der Lichtquelle bestimmt und insofern von der NA unabhängig. Hier ist es insbesondere möglich, die axiale und die transversale Auflösung durch die Festlegung der Bandbreite des durchstimmbaren Lasers und die apparative Vorgabe der numerischen Apertur unabhängig voneinander einzurichten. Besonders vorteilhaft ist es, beide Auflösungen etwa gleich groß einzurichten, wie man dies bei einer 3D-Bildgebung erwarten darf.

Der Vorteil der Erfindung ist nach dem zuvor Gesagten darin zu sehen, dass die Schwäche der Digitalen Holographie im Umgang mit hintereinander liegenden Streustrukturen durch eine eindimensionale spektrale Auswertung von simulierten Interferogrammen kompensiert und so überwunden wird. Da die Interferogramme nur simuliert werden und die Wahl der Rekonstruktionsebene frei ist, kann die Simulation in beliebigen anderen Probentiefen wiederholt werden. Aus der Sicht der OCT wird damit die Rayleigh-Längen-Begrenzung aufgehoben.

Hierin ist der wesentliche Gewinn des erfindungsgemäßen Verfahrens zu sehen: eine einzige optische Messung, bei der mehrere zweidimensionale Hologramme für ein Wellenlängenintervall aufgezeichnet werden, ist zugleich eine mit vorgegebener NA parallel an allen Messorten in einem vorbestimmten Probenoberflächenbereich durchgeführte FD-OCT-Messung über einen Messtiefenbereich in der Probe, der einem Vielfachen der Rayleigh-Länge entspricht.

Die Anwendbarkeit der OCT-Interpretation der Messdaten über viele Rayleigh-Längen hinweg ergibt sich aus der Propagierbarkeit der Wellenfelder, die sich aus den zweidimensional parallel aufgezeichneten Spektren rekonstruieren lassen. Das Referenzlicht erfüllt dabei zwei Funktionen zugleich: es dient der holographischen Rekonstruktion der Wellenfelder und der Laufzeitmessung des Lichts nach Art der OCT. Um aber die Laufzeiten in den Phasen der aus den Hologrammen berechneten und hiernach propagierten Lichtwellenfelder zur Auswertung der Tiefeninformation in der Umgebung der Rekonstruktionsebene verwenden zu können, ist eine Phasenanpassung der transversalen Raumfrequenzspektren erforderlich, welche die zwischen dem propagierten Wellenfeld und dem Referenzlicht entstehenden Laufzeitunterschiede kompensiert.

Die Messtiefe wird effektiv nur noch physikalisch begrenzt, wenn eben nicht mehr ausreichend auswertbares Licht aus der Probe auf den Detektor gelangt. Die Erfindung ermöglicht insoweit das optimale OCT-System, das mit einer - relativ schnellen - Messung alle überhaupt mittels OCT-Messung erreichbaren Tiefen eines vorgegebenen Probenvolumens gleichzeitig erfasst und durch numerische Auswertung einer 3D-Bildgebung zuführt. Die Rejustierung der Messvorrichtung und irgendwelche weiteren Messungen sind nicht mehr nötig.

Um dies vollständig ausnutzen zu können, muss man in der weiter oben stehenden Betrachtung einer Schicht die beliebig gewählte Koordinate $z = z_0$ durch eine andere Wahl ersetzen. Zum Beispiel ist es möglich, ohne dass es irgendeiner zusätzlichen Messung bedarf, dass der Datensatz $E_n(x, y, k_n = 2\pi/\lambda_n)$, $n = 1,..., N$ nicht nach $z_0$, sondern nach $z_0 + \Delta z$ propagiert wird, wobei

$$(8) \qquad \Delta z = \frac{\lambda_0}{\pi \mathrm{NA}^2}$$

mit $\lambda_0$ als Mittenwellenlänge des vorbestimmten Wellenlängenintervalls der durchstimmbaren Laserlichtquelle eine geeignete Wahl ist, da die zuvor berechnete Streustärkenverteilung in der Umgebung von $z = z0 + \Delta z$ unscharf und für die 3D-Bildgebung ungeeignet war. Erneut erfolgen dann die Propagation der Wellenfelder und die eindimensionale Fouriertransformation entlang $k$, und man erhält in analoger Weise wieder das Abbild einer Schicht, diesmal jedoch in einer anderen Tiefe der Probe. Dieses "schichtweise" Vorgehen kann fortgesetzt werden, bis alle messbaren Schichten der Probe analysiert und die Ergebnisse zu einem Volumendatensatz zusammengefügt worden sind.

Nachdem erklärt worden ist, wie sich die Streustärkenverteilung $S(x, y, z)$ für die gesamte Probe mit einer durch apparative Vorgaben in axialer und transversaler Richtung wählbaren Auflösung bestimmen lässt, ist die Aufgabe der Erfindung im Prinzip gelöst.

Allerdings ist insbesondere die transversale Auflösung nach dem bisher Gesagten nicht in allen Tiefen der Probe gleich gut, wenn die Dicken $\Delta z$ der berechneten Schichten zu groß gewählt sind. Die beste transversale Auflösung erhält man stets unmittelbar in der Rekonstruktionsebene, während darüber oder darunter Unschärfen infolge auseinanderlaufender Lichtstrahlen auftreten.

Dies ist jedoch kein Mangel des Messverfahrens, sondern ein Artefakt der numerischen Auswertung. Würde man die Streustärke für infinitesimal dünne Schichten berechnen, d. h. ließe man die Schichtdicken $\Delta z$ beliebig klein werden ($\Delta z \to 0$), so ergäbe sich die Streustärke $S(x, y, z)$ für jede Tiefe $z$ einzeln durch die Formel

$$(9) \qquad S(x,y,z) = \mathcal{F}_{2k \to z'}^{-1} \left[ \mathcal{F}_{2D}^{-1} e^{-ik(z+z_{\mathrm{ref}})} e^{ik_z(k,k_x,k_y)(z+z_{\mathrm{ref}})} \mathcal{F}_{2D}\left[ E(x,y;k) \right] \right]\Big|_{z'=z}$$

Die Rekonstruktionsdistanz $z_0$ aus (7) wird hierbei aufgeteilt in eine Distanz zu einer ersten Rekonstruktionsebene, $z_{\mathrm{ref}}$, und eine Distanz zwischen dieser ersten Rekonstruktionsebene und einer zweiten im Innern der Probe bei der Koordinate $z$, d. h. $z_0 = z + z_{\mathrm{ref}}$. (Erklärung s. u.)

Die numerische Auswertung der Formel (9) für jede feste Wahl der Koordinate $z$ würde leider einen enormen Rechenaufwand bedeuten. Dieser wäre vergleichbar mit den Arbeiten von Kim et al., bei denen eine rein holographische Rekonstruktion erfolgt. Der Vorteil der hier eingebundenen OCT-Auswertung wäre verloren.

Es ist jedoch besonders vorteilhaft, dass man in (9) die Reihenfolge der beiden zuletzt auszuführenden Fouriertransformationen vertauschen und somit die eindimensionale Integration über die axiale Richtung numerisch zuerst ausführen kann. Aus (9) wird dann:

$$
\begin{aligned}
S(x,y,z) &= \mathcal{F}_{2D}^{-1}\left[\mathcal{F}_{2k\to z}^{-1}\left[e^{-ik(z'+z_{\mathrm{ref}})}e^{+ik_z(k,k_x,k_y)(z'+z_{\mathrm{ref}})}\mathcal{F}_{2D}\left[E(x,y;k)\right]\right]\right]\\
&= \mathcal{F}_{2D}^{-1}\left[\int_{\mathbb{R}}\mathrm{d}\,(2k)\,e^{i2kz}e^{-ik(z+z_{\mathrm{ref}})}e^{ik_z(z+z_{\mathrm{ref}})}\mathcal{F}_{2D}\left[E(x,y;k)\right]\right]\\
&= 2\mathcal{F}_{2D}^{-1}\left[\int_{\mathbb{R}}\mathrm{d}k\,e^{i(k_z+k)z}\left(e^{i(k_z-k)z_{\mathrm{ref}}}\mathcal{F}_{2D}\left[E(x,y;k)\right]\right)\right].
\end{aligned}
\tag{10}
$$

Die Vertauschung der Fouriertransformationen impliziert eine Unterbrechung der Propagation in der ersten Rekonstruktionsebene. Die weitere Propagation wird zwar auch ausgeführt, aber sie erfolgt jetzt gleichzeitig im Zuge der eindimensionalen Fouriertransformation entlang der optischen Achse des Probenlichts mit der Berechnung der tiefenabhängigen Streustärken der gesamten Probentiefe. Dies macht die numerische Ausführung der eindimensionalen Fouriertransformation zwar etwas komplizierter, aber das damit berechenbare Ergebnis liefert eine so genannte "vollständige Rekonstruktion" der Probe für alle Messtiefen mit gleichbleibender Auflösung, ohne dass noch ein schichtweises Rekonstruieren erforderlich wäre. Bevorzugt wählt man für die vollständige Rekonstruktion die erste Rekonstruktionsebene in einem speziellen Abstand zur Kameraebene.

Für jedes OCT-Bild gibt es ein Messfenster, d. h. einen axialen Bereich in z (wenige Millimeter), der dargestellt werden kann. Dieser Bereich ist abhängig von der Breite des Wellenlängenintervalls und von der Sample-Rate der OCT-Kamera. Bei N erfassten Wellenlängen können N/2+1 Tiefenstützstellen diskriminiert werden. Das zugehörige Tiefenintervall wird i. a. begrenzt durch wenigstens eine Nyquist-Ebene. Die Nyquist-Ebene weist den maximalen Laufzeitunterschied des gestreuten Lichts zum Referenzlicht auf, der in Bezug auf das messtechnische Sampling noch erfasst werden kann, und ist somit eine Ebene in der Probe, aus dem Streuereignisse gerade noch eindeutig einer Tiefe zugeordnet werden können. Die zweite Begrenzung des Tiefenintervalls ist gewöhnlich die Referenz-Ebene, aus der gestreutes und zum 2D-Lichtdetektor-Array zurück geführtes Licht keinen Laufzeitunterschied zum Referenzlicht aufweist.

Es ist sehr vorteilhaft für die Ausführung der Integration gemäß Gleichung (10), die erste Rekonstruktionsebene als Nullebene der z-Achse einzurichten und dabei alle Streustrukturen der Probe auf derselben Seite der z-Achse zu wissen. Deshalb empfiehlt es sich, die zuvor genannte Referenz-Ebene, deren Lage relativ zur Probe am besten apparativ kontrolliert werden kann, als erste Rekonstruktionsebene zu wählen. Besonders bevorzugt ordnet man die Referenz-Ebene knapp außerhalb der Probe an, etwa durch die Einrichtung der apparativen Referenzarmlänge, wie es auch in der FD-OCT gängig ist. Es wäre im Prinzip auch möglich, die Nyquist-Ebene als erste Rekonstruktionsebene zu wählen, jedoch ist deren Lage schwierig zu definieren.

Zur Berechnung des Streuvermögens $S(x, y, z)$ muss die Integraltransformation $\int_{\mathbb{R}}dk e^{i(k_z+k)z}\,f(k)$ für eine Funktion $f(k)$ durchgeführt werden. Die numerische Berechnung des Integrals für den Datensatz $f(k_n)$, $n = 0,..., N - 1$ läßt sich schreiben als

$$
\sum_{n=0}^{N-1}e^{i\frac{2\pi}{N}(k_z(k_n,k_x,k_y)+k_n)z_m}f(k_n).
\tag{11}
$$

Die explizite Berechnung einer Fourier-Summe kann mittels einer Fast-Fourier-Transform (FFT) ausgeführt werden, doch effiziente Algorithmen hierfür setzen voraus, dass ein äquidistanter Satz von k-Stützstellen vorliegt. Genau dies trifft aber in (11) nicht zu. Man substituiert daher $\kappa_n = k_n + k_z$ mit $k_z^2 = k_n^2 - k_x^2 - k_y^2$, wobei $k_z$ selbst von $k_n$ abhängt und zusätzlich noch von den Pixelkoordinaten (x,y). Schreibt man $k_n$ als Funktion der unabhängigen Variablen $\kappa_n$, so findet man

$$
k_n = (\kappa_n^2 + k_x^2 + k_y^2)\,/\,(2\,\kappa_n),
\tag{12}
$$

und die Summe (11) ist dann schnell zu berechnen, wenn man die Funktion $f(k_n(\kappa_n))$ auf einen Satz von nicht-äquidistanten Stützstellen $k'_n$ mit der Eigenschaft (12) für einen äquidistanten Satz von $\kappa_n$ interpoliert. Die Integrationsaufgabe wird praktisch in eine Interpolationsaufgabe überführt. Eine geeignete Interpolation kann auf verschiedene Arten durchgeführt werden, die dem Fachmann geläufig sind, z.B. lineare Interpolation, Spline-Interpolation, Fourier-Interpolation, Sinc-Interpolation.

Die Integrationsaufgabe der Gleichung (10) wird durch Diskretisierung zu einer Summationsaufgabe gemäß (11), welche wiederum eine Fouriertransformation auf nicht-äquidistanten Stützstellen verlangt. Ein Algorithmus, der eine nicht-äquidistante Fouriertransformation durchführen kann, soll kurz als NFFT bezeichnet werden. Eine Möglichkeit der NFFT besteht wie dargelegt in einer FFT, die sich einer Interpolation der zu transformierenden Funktion auf die nicht-äquidistanten Stützstellen anschließt. Andere NFFT-Algorithmen, die womöglich auf Dateninterpolationt verzichten können, sind ebenso geeignet.

Durch die NFFT erfolgt eine numerische Berechnung von Streustärken auf grundsätzlich äquidistante Tiefenstützstellen, die auch als Rekonstruktionstiefen bezeichnet sein sollen. In einigen Fällen kann es vorkommen, dass sich der Abstand zwischen zwei Rekonstruktionstiefen $z_m$ und $z_{m+1}$ um einen bekannten Faktor verändert. Dies kann z. B. beim Einsatz von numerischen Vergrößerungen, die die axiale Rekonstruktionsdistanz verändern, oder bei der Rekonstruktion von Streuungen in Medien mit Brechungsindex $n$ passieren. In diesem Fall können zur Abhilfe die $\kappa_n$-Achsen (zur Verdeutlichung: jeder Pixel (x,y) besitzt eine eigene $\kappa_n$-Achse) mit dem Kehrwert des besagten Faktors skaliert werden, um die räumliche Längenänderung numerisch zu kompensieren. Praktisch sind dann etwa die Interpolationen auf einem skalierten Stützstellensatz vorzunehmen.

Falls der Brechungsindex der Probe sogar über die Messtiefe variiert, dann variieren auch die Abstände der Rekonstruktiostiefen über die Messtiefe. Dies kann dann nur noch über eine Fouriertransformation mit nicht-äquidistanten Stützstellen im Wellenzahl- und auch im Tiefenbereich (kurz: NNFFT) kompensiert werden, um weiterhin eine scharfe Abbildung in allen Messtiefen zu gewährleisten.

Zusammenfassend stellt sich die erfindungsgemäße Auswertung wie folgt dar:

1. Der aufgenommene Datensatz (Kamerabilder) $I_n(x, y, k_n)$ wird durch Kenntnis der Referenzwelle in komplexe Wellenfelder $E_n(x, y, k_n)$ zurückgerechnet. Andere Terme (Rauschen, DC-Anteil, konjugiertes Feld) werden eliminiert oder vernachlässigt.
2. Man berechnet die zweidimensionale Fourier-Transformation (d. h. die Ebene-Wellen-Zerlegung bzw. das Angular Spectrum) $\tilde{E}(k_x, k_y, k_n)$ der einzelnen Wellenfelder $E_n(x, y, k_n)$.
3. Die so enstandenen Datensätze werden zur Referenzebene propagiert indem alle Datenpunkte $\tilde{E}(k_x, k_y, k_n)$ mit $e^{i(k_z(k_n,k_x,k_y)-k_n)z_{ref}}$ multipliziert werden. Dieser Schritt umfaßt neben der Propagation auch die Phasenkorrektur zur Wahrung der Laufzeitunterschiede zwischen Proben- und Referenzlicht, den Faktor $\exp(-ik_n z_{ref})$.
4. Auf den so erhaltenen Daten $\tilde{E}(k_x, k_y, k_n)$ wird eine NFFT mit den durch $k_z(k_n, k_x, k_y) + k_n$ gegebenen Stützstellen durchgeführt. Man erhält das Angular-Spectrum für alle Tiefen, d. h. $\tilde{E}(k_x, k_y, z_m)$.
5. Die so berechneten Daten $\tilde{E}(k_x, k_y, z_m)$ werden zurück in den Raumbereich transformiert (inverse zweidimensionale Fourier-Transformation entlang der $k_x$ und $k_y$ Achsen).

Das zunächst zu unterstellende Problem der Beiträge von Mehrfachstreuungen, die in der OCT durch konfokale Abbildungen unterdrückt werden, hat sich in der Praxis als nicht tiefgreifend erwiesen. Zwar treten diese Mehrfachstreuungen auf, aber sie führen erst bei hohen Messtiefen in der Probe zur deutlichen Degradierung der gewonnenen Strukturdaten.

Mit den Mitteln der Erfindung ist es ohne Weiteres möglich, durch die Aufnahme eines einzigen Satzes von Hologrammen im vorbestimmten Wellenlängenintervall - in wenigen Sekunden oder gar in Bruchteilen einer Sekunde - gute dreidimensionale Strukturinformationen über einen Tiefenbereich von mehr als zehn Rayleigh-Längen zu gewinnen.

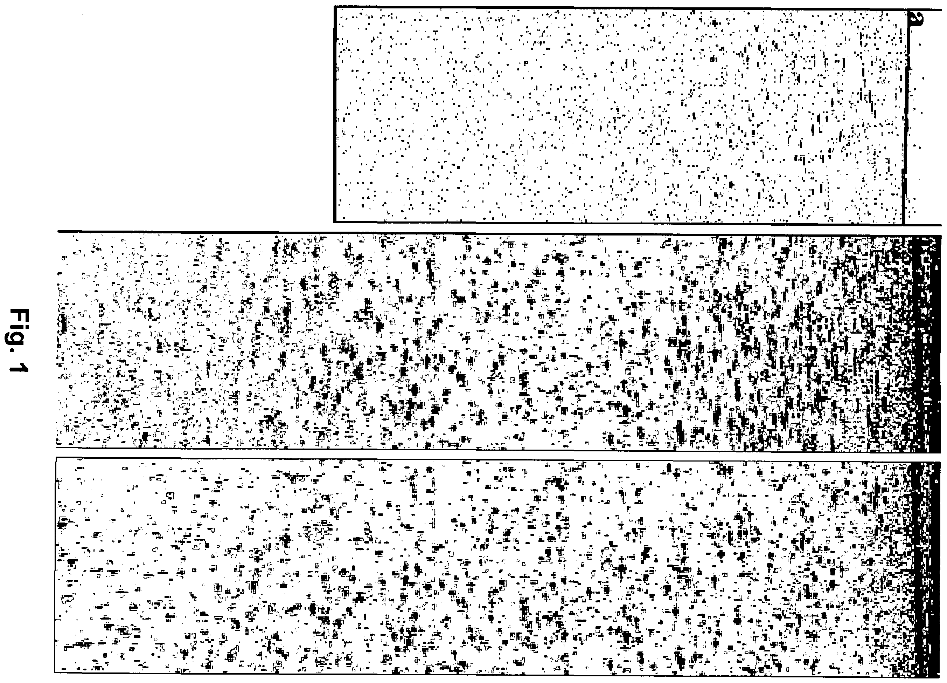

Um dies zu illustrieren, werden nun noch Abbildungen von Messergebnissen vorgestellt: Fig. 1 zeigt invertiert dargestellte Tomogramme einer synthetischen Streuprobe (P.D. Wooliams, R.A. Ferguson, C. Hart, A. Grimwood, P.H. Tomlins, App. Optics, Vol. 49, No. 11, 2014-2021, 2010) bestehend aus Polyurethan Harz in dem sphärische rote Eisenoxid Nanopartikel eingebettet wurden, welche einen Durchmesser zwischen 300 - 800 nm aufweisen. Fig.1 a) zeigt das Ergebnis einer Messung der Streuprobe mit einem konfokal scannenden OCT mit einer NA von ca. 0,06. Im Bereich des konfokalen Fokus, sind die einzelnen Streuer klar zu sehen. Außerhalb nehmen die Auflösung und auch die Intensität der Streuer ab. Eine Messung nach dem erfindungsgemäßen Verfahren mit einer NA von ca. 0,07 und unter Rekonstruktion einer einzelne Probenschicht ist in Fig. 1 b) zu sehen. In der Rekonstruktionsdistanz ist die Auflösung der Streuprobe beugungsbegrenzt und nimmt außerhalb erwartungsgemäß ab. Die Intensität ist im Gegensatz zu Fig. 1 a) nicht durch ein konfokales Gating begrenzt. Die Messung in Fig. 1 c) ist identisch zu der Messung aus b), allerdings wird diesmal der gesamte Tiefenbereich über eine NFFT-Auswertung rekonstruiert. Es ist erkennbar, dass die Auflösung über mehrere Rayleigh-Längen beugungsbegrenzt ist. Weder Intensität noch Auflösung zeigen Variationen über die Tiefenachse (in allen Bildern von oben nach unten verlaufend) der Probe.

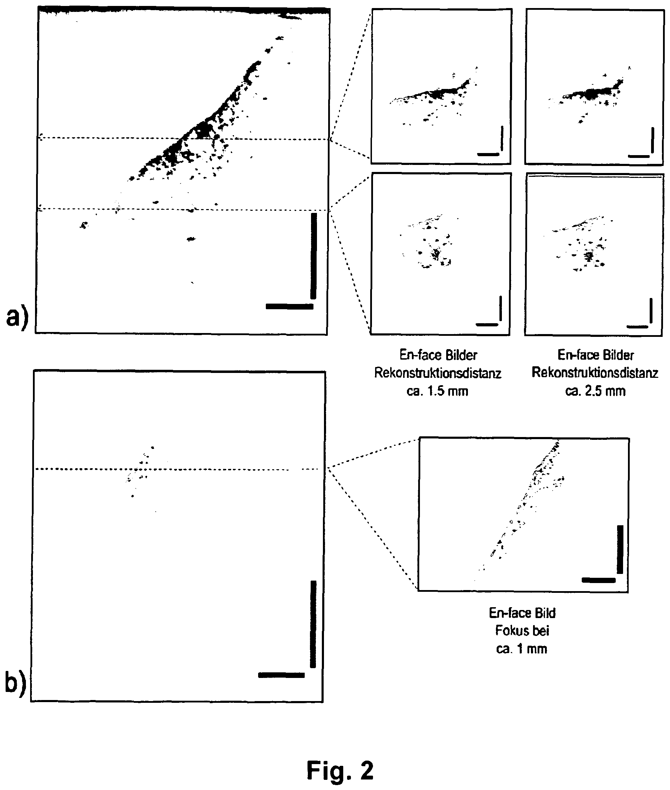

Fig.2 zeigt invertiert dargestellte Tomogramme einer schräggestellten Weintraube. Es wurde jeweils eine Aufnahme eines erfindungsgemäß ausgebildeten Messsystems mit einer NA von 0,07 (a) mit einem konfokalen OCT System mit einer NA von 0,06 (b) verglichen. Die Rekonstruktionsdistanz wird variiert, und es sind en face Bilder für zwei Schnitte mit einer Rekonstruktionsdistanz von 1.5 mm, bzw. 2.5 mm gezeigt. Der Fokus des konfokalen OCT ist bei 1 mm Tiefe fest vorgegeben. Der Intensitätsverlust außerhalb der Rayleigh-Länge beim konfokalen OCT ist deutlich zu sehen. Im Gegensatz dazu sind in den Abbildung der Fig. 2 a) streuende Strukturen deutlich über die Rayleigh-Länge hinaus zu erkennen. In den en face Bildern kann man die Auswirkung durch unterschiedliche Rekonstruktionsdistanzen beobachten. Alle eingezeichneten Skalen sind 1 mm.

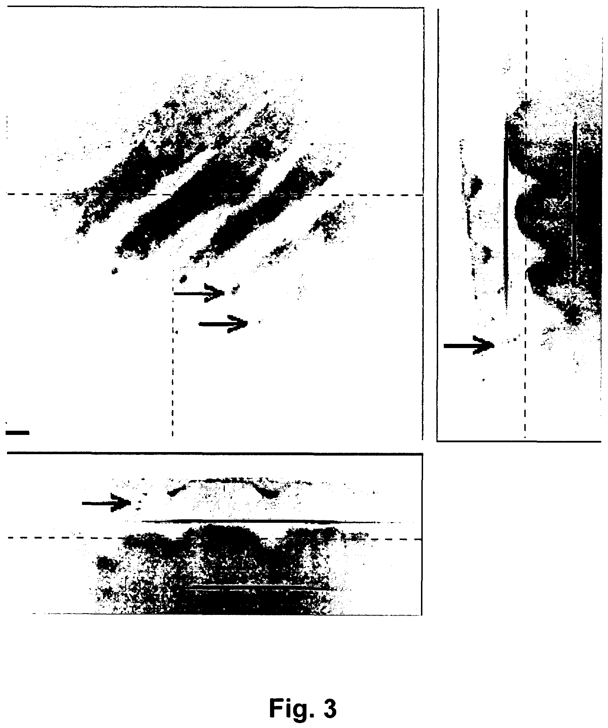

In Fig. 3 sind drei senkrechte Schnitte durch eine *in vivo* gemessene menschliche Fingerspitze dargestellt (invertierte Darstellung). Die Messung erfolgte mit dem hier beschriebenen Verfahren zur Bestimmung der gesamten Streustärkenverteilung binnen weniger Sekunden. Bei der Auswertung wurde eine Rekonstruktionsebene in die Ebene der Schweißdrüsen gelegt. Die horizontalen Linien im Bild sind Artefakte die durch Reflexionen an einer Glasplatte vor dem Sensor der Kamera entstehen. Die gestrichelten Linien zeigen die jeweilige Lage der anderen Schnitte.

Die Erfindung kann im Prinzip mit einer beliebigen Vorrichtung zur Digitalen Holographie und einer durchstimmbaren Lichtquelle durchgeführt werden. Eine hohe Durchstimmgeschwindigkeit der Lichtquelle und eine sehr hohe Aufnahmegeschwindigkeit der elektronischen Kamera sind sehr empfehlenswert, insbesondere bei der in-vivo Anwendung.

Ergänzend soll noch auf die Möglichkeiten zur Kompensation von Bewegungsartefakten in der Numerik eingegangen werden.

Für in-vivo Aufnahmen ist bei den momentanen Geschwindigkeiten der digitalen Kameras ein Problem, dass sich das Objekt häufig während der Aufnahme bewegt, was zu Artefakten in den Tomogrammen führt. Hierbei sind Bewegungen in *z*-Richtung, d. h. entlang der optischen Achse am problematischsten, da sich zusätzlich zu der eigentlich physischen Bewegungen durch den Dopplereffekt Verschiebungen des rekonstruierten Objektes ergeben. Die *z*-Bewegungen haben zudem die Eigenschaft, die Laufzeit des Lichtes für unterschiedliche Wellenzahlen zu verändern, was in den Aufnahmen zu Effekten führt, die nicht von Dispersion zu unterscheiden sind.

Bewegungen lassen sich durch eine Zeit-Frequenz-Analyse des über die Wellenzahl k gemessenen Signals bestimmen, da eine zeitlich variierende Bewegungen über die Doppler-Verschiebung das Signal für alle sich gleich bewegende Volumenanteile charakteristisch verändert. Hierzu bietet sich beispielsweise eine "Short-Time" Fourier-Transformation (STFT) an mit der man globale Bewegungen des Objektes nachvollziehen kann: Man fenstert einen bestimmten Wellenzahlbereich, der einem Zeitintervall entspricht, welches kürzer als die gesamte Aufnahmezeit ist. Dies wird i. a. die Auflösung nach der Rekonstruktion verringern, allerdings ist das Objekt jetzt nicht mehr so stark durch Bewegungsartefakte gekennzeichnet da die Fensterung eine kürzere Aufnahmezeit selektiert hat. Führt man dies für mehrere zentrale Wellenzahlen und damit Messzeitpunkte aus, so kann man in den einzeln rekonstruierten Tomogrammen die Bewegungen erkennen und bestimmen. Prinzipiell kann man diese Information mit allen Transformationen extrahieren, welche eine Zeit-Frequenz-Darstellung des Signals erlauben (STFT, Wavelet-Transformation, Gabor-Transformation, etc.). Für die Extraktion der Bewegung aus der Zeit-Frequenz-Darstellung kann man beispielsweise eine Kreuzkorrelation der Kurzzeitbilder durchführen.

Ist die Bewegung bekannt, so lässt sie sich der Effekt der Bewegung durch Multiplikation mit zu den Bewegungen gehörenden inversen Phasen im Fourierraum des Tomogrammes wieder rückgängig machen. Dabei ist zu beachten, dass eine Phasenänderung um $\exp(i\phi(k))$ durch die STFT eine Verschiebung der Einzelbilder um $\partial_k\phi(k)$ verursacht:

$$(13)$$

$$
\begin{aligned}
\mathrm{STFT}_{k_0}\left[\tilde{f}(k) \cdot \exp\left(\mathrm{i}\phi(k)\right)\right] &= \int_{k_0-\Delta k}^{k_0+\Delta k} \mathrm{d}k\, \tilde{f}(k)\exp\left(\mathrm{i}\left(kz+\phi(k)\right)\right) \\
&= \int_{k_0-\Delta k}^{k_0+\Delta k} \mathrm{d}k\, \tilde{f}(k)\exp\left[\mathrm{i}\left(kz+\phi(k_0)+k\partial_k\phi(k)|_{k=k_0}+\mathcal{O}(k^2)\right)\right] \\
&\approx \exp\left(\mathrm{i}\phi(k_0)\right)\cdot\tilde{f}\left(z+\partial_k\phi(k)|_{k=k_0}\right)
\end{aligned}
$$

Folglich muss die aus der Kreuzkorrelation bestimmte Verschiebung der Kurzzeitbilder integriert werden um eine geeignete Phasenkompensation zu finden.

Es kann sich unterschiedliche Gruppengeschwindigkeitsdispersion in Proben- und Referenzarm zeigen, wenn man einen asymmetrischen Aufbau von Proben- und Referenzarm wählt, wie z.B. bei einem Mach-Zehnder-Interferometer. Dies führt zu einer Verringerung von Auflösung und Signalstärke. Mathematisch zeigen sich die Dispersionsunterschiede in einer zusätzlichen von der Wellenzahl bzw. Wellenlänge abhängigen Phase, d. h. das Spektrum $S(\lambda)$ ist gegeben durch

$$(14) \qquad S(\lambda) = 2\mathrm{Re}\int \mathrm{d}z\, f(z)\exp\left(\mathrm{i}k(\lambda)z+\mathrm{i}\phi(\lambda)\right)+\dots,$$

wobei $\phi(\lambda)$ durch die Dispersion bestimmt ist. Die Phase kann kompensiert werden, indem man die Daten vor der Fouriertransformation entlang $k$, bzw. entlang $k_z$ mit einer geeigneten von der Wellenlänge abhängigen Phasenfunktion multipliziert wird, d.h. mit $\exp(-i\phi(\lambda))$. Auf Grund der identischen Auswirkung von Dispersion und Bewegung des Objektes in axialer Richtung während der Messung, kann diese Phasenfunktion mit dem gleichen Algorithmus wie bei der Bewegungskorrektur bestimmt werden. Da die Gruppengeschwindigkeitsdispersion in der Regel konstant ist, muss die Bestimmung der Phasenfunktion nicht für jede Messung wiederholt werden.

Experimentell kann die Bestimmung der Dispersion durch Aufnahme von zwei Spiegeln in unterschiedlichen Tiefen erfolgen. Hieraus kann man mittels Hilbert-Transformationen die korrekten Phasenmultiplikatoren bestimmen. Sind zwei Spiegel in den zur Referenz relativen Tieferen $z_1$ und $z_2$ gegeben, so sind die dazugehörigen Interferenzsignale $S(\lambda)$ und $S_2(\lambda)$ gegeben durch

$$S_1(\lambda) = 2\mathrm{Re}\int_{-\infty}^{+\infty} dz\, \delta(z-z_1)\cdot\exp(ik(\lambda)z_1 + i\phi(\lambda)) = 2\cos\left(k(\lambda)z_1 + \phi(\lambda)\right)$$

$$(15) \qquad S_2(\lambda) = 2\mathrm{Re}\int_{-\infty}^{+\infty} dz\, \delta(z-z_2)\cdot\exp(ik(\lambda)z_2 + i\phi(\lambda)) = 2\cos\left(k(\lambda)z_2 + \phi(\lambda)\right)$$

Vernachlässigt man zusätzliche Terme (DC Terme, Autokorrelationen, keine weiteren Signale, etc.) und ist $z_1 > 0$ und $z_2 > 0$ so ist die Hilbert-Transformation dieser Signale gegeben durch

$$(16) \qquad \begin{aligned} S_1 + i\mathcal{H}\left[S_1(\lambda)\right] &= 2\exp\left(ik(\lambda)z_1 + i\phi(\lambda)\right) \\ S_2 + i\mathcal{H}\left[S_2(\lambda)\right] &= 2\exp\left(ik(\lambda)z_2 + i\phi(\lambda)\right) \end{aligned}$$

Mit Kenntnis der Spiegelpositionen $z_1$ und $z_2$, die man aus den rekonstruierten Daten (vergleichbar einem OCT A-scan) gewinnen kann, lassen sich die benötigte Phase $\phi(\lambda)$ und $k(\lambda)$ auflösen:

$$k(\lambda) = -\frac{1}{(z_1 - z_2)}\left[\arg\left(S_1(\lambda) + \mathcal{H}\left[S_1(\lambda)\right]\right) - \arg\left(S_2(\lambda) + \mathcal{H}\left[S_2(\lambda)\right]\right)\right]$$

$$(17) \qquad \phi(\lambda) = \frac{1}{(z_1 - z_2)}\left[z_2 \arg\left(S_1(\lambda) + \mathcal{H}\left[S_1(\lambda)\right]\right) - z_1 \arg\left(S_2(\lambda) + \mathcal{H}\left[S_2(\lambda)\right]\right)\right]$$

Hierbei muss $\lambda$ nicht notwendigerweise die Wellenlänge darstellen, sondern ist die Variable in der zeitlich linear gesweept wurde, d. h. $\lambda(t) = \lambda_0 + \lambda_1 t$.

Für dieses Verfahren wird allerdings lediglich eine Spiegelaufnahme mit nur einer Tiefe benötigt, wenn entweder keine Dispersion vorhanden ist, also $\phi(\lambda(k)) = \phi_0 + \phi_1 k$ mit konstanten $\phi_0$ und $\phi_1$ oder wenn linear in $k$ gesweept wurde, also wenn $k(\lambda) = k_0 + k_1\lambda$ mit konstanten $k_0$ und $k_1$.

Zusammenfassend soll hervorgehoben werden, dass sich Artefakte in den Laufzeitunterschieden zwischen Referenz- und Probenlicht durch Gruppengeschwindigkeitsdispersion oder durch eine Translation (z.B. Eigenbewegung) der Probe ergeben können, die sich durch eine weitere Phasenmultiplikation der transversalen Raumfrequenzspektren aber kompensieren lassen. Wichtig ist dabei, dass die Phasenfaktoren, die selbst von den $k_n$ abhängen, vor der eindimensionalen Fouriertransformation zur Berechnung der Streustärken eingeführt werden müssen.

**Patentansprüche**

1. Verfahren zur 3D-Bildgebung einer streuenden Probe, insbesondere zur Bestimmung der räumlichen Streustärkenverteilung S(x,y,z) der Probe, mit den Schritten

a) Aufteilen von Laserlicht mit innerhalb einer vorgegebenen Bandbreite wählbarer Wellenlänge in einen Proben- und einen Referenzlichtstrahl,
b) Beleuchten der Probe mit N verschiedenen Wellenlängen $\lambda_n$, n = 1, ..., N und Rückführen des in der Probe gestreuten Lichts sowie des Referenzlichts auf ein 2D-Lichtdetektor-Array,
c) Überlagern des gestreuten Probenlichts mit dem Referenzlicht auf dem 2D-Lichtdetektor-Array und Aufzeichnen je eines Hologramms für jede der N Wellenlängen,

d) Berechnen des in der Probe gestreuten elektrischen Wellenfeldes des Probenlichts für jede der N Wellenlängen in der Ebene des 2D-Lichtdetektor-Arrays,

e) Propagieren, mittels eines Propagationskerns des Probenlichts, aller berechneten Wellenfelder des Probenlichts in eine vorbestimmte Rekonstruktionsebene, die senkrecht zur optischen Achse des Probenlichtstrahls ausgerichtet ist,

f) Berechnen des transversalen Raumfrequenzspektrums $E_n(k_x,k_y,k_n)$, mit $k_n = 2\pi/\lambda_n$ des in der Probe gestreuten und in die Rekonstruktionsebene propagierten elektrischen Wellenfeldes für jede der N Wellenlängen,

**gekennzeichnet durch** die weiteren Schritte:

g) Phasenanpassung des transversalen Raumfrequenzspektrums für jede der N Wellenlängen des elektrischen Wellenfeldes des Probenlichts zur Kompensation der durch Schritt d) und/oder Schritt e) zwischen dem berechneten Wellenfeld des Probenlichts und dem Wellenfeld des Referenzlichts entstehenden Laufzeitunterschiede, wobei ein durch Schritt d) eingeführter Laufzeitunterschied durch eine Multiplikation der Referenzwelle $R(x, y; k)$ des Referenzlichts mit einem Phasenfaktor kompensiert wird, wobei der Phasenfaktor so gewählt wird, dass die resultierende Referenzwelle $R'(x, y; k)$ für ein Koordinatenpaar $(x, y)$ nicht mehr von der Wellenzahl $k$ abhängt und/oder ein durch Schritt e) eingeführter Laufzeitunterschied durch eine Multiplikation des Propagationskerns mit einem Phasenfaktor kompensiert wird, wobei der Phasenfaktor so gewählt wird, dass der Propagationskern für eine feste transversale Raumfrequenz $(k_x, k_y)$ unabhängig von der Wellenzahl $k$ ist,

h) Berechnung des tiefenabhängigen Streustärkespektrums $S(k_x,k_y,z)$ durch eindimensionale Fouriertransformation der Raumfrequenzspektren für jede transversale Raumfrequenz $(k_x, k_y)$ entlang der optischen Achse des Probenlichtstrahls,

i) Berechnen der Streustärke $S(x,y,z)$ wenigstens in einer Schicht der Probe in der Umgebung der Rekonstruktionsebene.

2. Verfahren nach Anspruch 1, **dadurch gekennzeichnet, dass** die Rekonstruktionsebene im Innern der Probe vorbestimmt wird.

3. Verfahren nach Anspruch 2, **dadurch gekennzeichnet, dass** die Berechnung der Streustärke $S(x,y,z)$ für eine Mehrzahl von entlang der optischen Achse des Probenlichtstrahls beabstandeten Rekonstruktionsebenen bzw. Schichten der Probe erfolgt, und diese Ergebnisse im Rechner zu einer Streustärkeverteilung für die gesamte Probe zusammengefügt werden.

4. Verfahren nach einem der Ansprüche 1 oder 2, **dadurch gekennzeichnet, dass** die Rekonstruktionsebene derart vorbestimmt wird, dass in der Rekonstruktionsebene gestreutes und zum 2D-Lichtdetektor-Array zurück geführtes Licht keinen Laufzeitunterschied zum Referenzlicht aufweist.

5. Verfahren nach einem der vorangehenden Ansprüche, **dadurch gekennzeichnet, dass** die Phasenanpassung dadurch erfolgt, dass jedes transversale Raumfrequenzspektrum mit einem Phasenfaktor abhängig von $k_n$ multipliziert wird, so dass die Komponenten $E_n(k_{x0}, k_{y0}, k_n)$ aller N Raumfrequenzspektren in der Rekonstruktionsebene für eine vorbestimmte transversale Raumfrequenz $(k_{x0}, k_{y0})$ denselben Wert annehmen.

6. Verfahren nach einem der vorangehenden Ansprüche **gekennzeichnet durch** numerische Kompensation von Artefakten durch Probenbewegung und/oder Gruppengeschwindigkeitsdispersion durch Multiplikation aller transversalen Raumfrequenzspektren mit einem vom jeweiligen $k_n$ abhängigen Phasenfaktor vor der Durchführung der eindimensionalen Fouriertransformation.

7. Verfahren nach einem der Ansprüche 4 oder 5, **dadurch gekennzeichnet, dass** die eindimensionale Fouriertransformation als Fast Fourier Transform (FFT) über einen von der jeweiligen Raumfrequenz $(k_x, k_y)$ abhängigen, nicht-äquidistanten Stützstellensatz ausgeführt wird, wobei die Raumfrequenzspektren zuvor auf diese Stützstellen interpoliert werden.

8. Verfahren nach einem der vorangehenden Ansprüche, **dadurch gekennzeichnet, dass** im Referenzarm ein strahlaufweitendes Element vorgesehen ist, welches das Referenzlicht vor dem Eintreffen auf dem 2D-Lichtdetektor-Array sphärisch aufweitet.

9. Verfahren nach Anspruch 8, **dadurch gekennzeichnet, dass** das Referenzlicht einen Fokus in der Nähe des zu untersuchenden Probenbereichs außerhalb der Probe aufweist.

10. Verfahren nach einem der vorangehenden Ansprüche, **dadurch gekennzeichnet, dass** mehr als N = 100 verschiedene Wellenlängen verwendet werden.

**Claims**

1. A method for 3D imaging of a scattering sample, in particular for determining the spatial scattering-power distribution S(x,y,z) of the sample, having the following steps

   a) splitting laser light having a wavelength that can be selected within a predetermined band width, into a sample and a reference light beam,
   b) illumining the sample at N different wavelengths $\lambda_n$, $n = 1,..., N$, and returning the light scattered in the sample and also the reference light onto a 2D light detector array,
   c) superposing the scattered sample light with the reference light on the 2D light detector array and recording in each case one hologram for each of the N wavelengths,
   d) calculating the electric wave field of the sample light scattered in the sample for each of the N wavelengths in the plane of the 2D light detector array,
   e) propagating, using a propagation core to the sample light, all calculated wave fields of the sample light, in a predetermined reconstruction plane that is aligned perpendicular to the optical axis of the sample light beam,
   f) calculating the transverse space-frequency spectrum $E_n (k_x, k_y, k_n)$, with $k_n = 2\pi/\lambda_n$ of the electric wave field scattered in the sample and propagated in the reconstruction plane for each of the N wavelengths,

   **characterized by** the further steps:

   g) phase matching of the transverse space-frequency spectrum for each of the N wavelengths of the wave fields of the sample light for compensation of the propagation-time differences produced by steps d) and/or step e) between the calculated wave field of the sample light and wave field of the reference light, wherein a propagation time difference introduced in step d) is compensated by multiplication of the reference wave $R(x; y; k)$ of the reference light with a phase factor, wherein the phase factor is selected so that the resulting reference wave $R'(x; y; k)$ for a coordinate pair $(x,y)$ no longer depends on the wave number $k$ and/or a propagation time difference introduced in step e) is compensated by multiplication of the propagation core with a phase factor, wherein the phase factor is selected so that the propagation core is independent from the wave number k for a fixed transverse spatial frequency $(kx,ky)$,
   h) calculating the depth-dependent scattering-power spectrum $S(kx,ky,z)$ by one-dimensional Fourier transformation of the space-frequency spectra for each transverse space frequency $(kx,ky)$ along the optical axis of the sample beam,
   i) calculating the scattering power $S(x,y,z)$ in at least one layer of the sample in the surroundings of the reconstruction plane.

2. The method according to claim 1, **characterized in that** the reconstruction plane is predetermined inside the sample.

3. The method according to claim 2, **characterized in that** the calculation of the scattering power $S(x,y,z)$ takes place for a plurality of reconstruction planes or layers of the sample spaced along the optical axis of the sample light beam and these results are combined in the computer to a scattering-power distribution for the entire sample.

4. The method according to one of claims 1 or 2, **characterized in that** the reconstruction plane is predetermined in such a manner that light scattered in the reconstruction plane and returned to the 2D light detector array exhibits no propagation-time difference to the reference beam.

5. The method according to one of the preceding claims, **characterized in that** phase matching takes place by multiplying each transverse space-frequency spectrum by a phase factor as a function of $k_n$ so that the components $E_n$ $(k_{x0},k_{y0},k_n)$ of all N space-frequency spectra in the reconstruction plane assume the same value for a predetermined transverse space frequency $(k_{x0},k_{y0})$.

6. The method according to one of the preceding claims, **characterized by** numeric compensation of artifacts by sample movement and/or group velocity dispersion by multiplication of all transverse space-frequency spectra by a phase factor in each case dependent on the respective $k_n$ prior to carrying out the one-dimensional Fourier transformation.

7. The method according to one of claims 4 or 5, **characterized in that** the one-dimensional Fourier transformation is carried out as a Fast Fourier Transform (FFT) across a non-equidistant supporting-point set that is dependent on the respective space frequency (*kx,ky*), the space-frequency spectra being previously interpolated onto these supporting points.

8. The method according to one of the preceding claims, **characterized in that** a beam-spreading element is provided in the reference arm that spreads spherically the reference light before it impinges on the 2D light detector array.

9. The method according to claim 8, **characterized in that** the reference light exhibits a focus in the proximity of the sample area to be investigated outside the sample.

10. The method according to one of the preceding claims, **characterized in that** not fewer than N = 100 different wavelengths are used.

**Revendications**

1. Procédé d'imagerie 3D d'un échantillon de diffusion, plus particulièrement pour la détermination de la répartition spatiale des intensités de diffusion S(x,y,z) de l'échantillon, avec les étapes suivantes

   a) division d'une lumière laser avec une longueur d'onde pouvant être choisie à l'intérieur d'une largeur de bande déterminée en un faisceau lumineux d'échantillon et un faisceau lumineux de référence,
   b) éclairage de l'échantillon avec N différentes longueurs d'ondes $\lambda_n$, n = 1..., N et retour de la lumière diffusée dans l'échantillon ainsi que de la lumière de référence vers une matrice de photodétecteurs 2D,
   c) superposition de la lumière diffusée par l'échantillon avec la lumière de référence sur la matrice de photodétecteurs 2D et enregistrement d'un hologramme pour chacune des N longueurs d'ondes,
   d) calcul du champ d'onde électrique diffusé dans l'échantillon de la lumière de l'échantillon pour chacune des N longueurs d'ondes dans le plan de la matrice de photodétecteurs 2D,
   e) propagation, au moyen d'un noyau de propagation de la lumière de l'échantillon, de tous les champs d'ondes de la lumière de l'échantillon vers un plan de reconstruction prédéterminé, qui est orienté perpendiculairement à l'axe optique du faisceau lumineux de l'échantillon,
   f) calcul du spectre de fréquence spatial transversal $E_n$ ($k_x$, $k_y$, $k_z$) avec $k_n = 2\pi/\lambda n$ du champ d'onde électrique diffusé dans l'échantillon et propagé vers le plan de reconstruction pour chacune des N longueurs d'ondes,

   **caractérisé par** les étapes supplémentaires :

   g) adaptation de la phase du spectre de fréquence spatial transversal pour chacune des N longueurs d'ondes du champ d'ondes électrique de la lumière de l'échantillon pour la compensation des différences de temps de parcours apparaissant, du fait de l'étape d) et/ou de l'étape e), entre le champ d'ondes calculé de la lumière de l'échantillon et le champ d'ondes de la lumière de référence, une différence de temps de parcours introduite par l'étape d) étant compensée par une multiplication de l'onde de référence R(x,y,z) de la lumière de référence par un facteur de phase, le facteur de phase étant choisi de façon à ce que l'onde de référence R'(x,y,z) qui en résulte pour une paire de coordonnées (x,y) ne dépende plus du nombre d'onde k et/ou une différence de temps de parcours introduite par l'étape e) étant compensée par une multiplication du noyau de propagation par un facteur de phase, le facteur de phase étant choisi de façon à ce que le noyau de propagation pour une fréquence spatiale transversale fixe (kx, ky) soit indépendant du nombre d'onde k,
   h) calcul du spectre d'intensités de diffusion en fonction de la profondeur S(kx, ky, kz) par une transformation de Fourier monodimensionnelle pour chaque fréquence spatiale transversale (kx, ky) le long de l'axe optique du faisceau lumineux de l'échantillon,
   i) calcul de l'intensité de diffusion S(x,y,z) au moins dans une couche de l'échantillon dans l'environnement du plan de reconstruction.

2. Procédé selon la revendication 1, **caractérisé en ce que** le plan de reconstruction est prédéterminé à l'intérieur de l'échantillon.

**3.** Procédé selon la revendication 2, **caractérisé en ce que** le calcul de l'intensité de diffusion S(x,y,z) a lieu pour une pluralité de plans de reconstruction distants le long de l'axe optique du faisceau lumineux de l'échantillon ou de couches de l'échantillon et ces résultats sont assemblés dans l'ordinateur en une répartition des intensités de diffusion pour l'ensemble de l'échantillon.

**4.** Procédé selon l'une des revendications 1 ou 2, **caractérisé en ce que** le plan de reconstruction est prédéterminé de façon à ce que la lumière diffusée dans le plan de reconstruction et retournée vers la matrice de photodétecteurs 2D ne présente aucune différence de temps de parcours par rapport à la lumière de référence.

**5.** Procédé selon l'une des revendications précédentes, **caractérisé en ce que** l'adaptation de la phase a lieu grâce au fait que chaque spectre de fréquence spatiale transversal est multiplié par un facteur de phase dépendant de $k_n$ de façon à ce que les composantes $E_n (k_{xo}, k_{y0}, k_n)$ de tous les N spectres de fréquences spatiales dans le plan de reconstruction adoptent, pour une fréquence spatiale transversale $(k_{x0}, k_{y0})$ prédéterminée, la même valeur.

**6.** Procédé selon l'une des revendications précédentes, **caractérisé par** une compensation numérique d'artefacts par le déplacement de l'échantillon et/ou une dispersion de vitesses de groupes par la multiplication de tous les spectres de fréquences spatiales transversales par un facteur de phase dépendant de $k_n$ avant la réalisation de la transformation de Fourier monodimensionnelle.

**7.** Procédé selon l'une des revendications 4 ou 5, **caractérisé en ce que** la transformation de Fourier monodimensionnelle est exécutée comme une Fast Fourier Transforme (FFT) sur un ensemble de points d'appui non équidistants dépendant de la fréquence spatiale $(k_x, k_y)$ correspondante, les spectres de fréquences spatiales étant auparavant interpolés sur ces points d'appui.

**8.** Procédé selon l'une des revendications précédentes, **caractérisé en ce que**, dans le bras de référence, un élément d'élargissement de faisceau est prévu, qui élargit la lumière de référence de manière sphérique avant l'arrivée sur la matrice de photodétecteurs 2D.

**9.** Procédé selon la revendication 8, **caractérisé en ce que** la lumière de référence présente un point focal à proximité de la zone de l'échantillon à examiner à l'extérieur de l'échantillon.

**10.** Procédé selon l'une des revendications précédentes, **caractérisé en ce que** plus de N = 100 différentes longueurs d'ondes sont utilisées.

**Fig. 1**

En-face Bilder
Rekonstruktionsdistanz
ca. 1.5 mm

En-face Bilder
Rekonstruktionsdistanz
ca. 2.5 mm

En-face Bild
Fokus bei
ca. 1 mm

**Fig. 2**

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7602501 B2

**In der Beschreibung aufgeführte Nicht-Patentliteratur**

- **L. YU ; M.K. KIM.** *Wavelength-Scanning Digital Interference Holography*
- Variable tomographic scanning with wavelength scanning digital interference holography. *Optics Communications,* 2006, vol. 260, 462-468
- **M.K. KIM.** Principles and techniques of digital holographic Microscopy. *SPIE Reviews 018005-1,* 2010, vol. 1
- Optical tomography for biomedical applications by digital interference holography. **POTCOAVA et al.** Measurement Science and Technology. IOP, 01. Juli 2008, vol. 19, 7-4010, 1-7 4010, 8
- **BLAZKIEWICZ et al.** *Experimental Demonstration of Signal-to-Noise-Ratio Improvement of Fourier-Domain Optical Coherence Tomography*
- **P. BLAZKIEWICZ ; M. GOURLAY ; J. R. TUCKER ; A. D. RAKIC ; A. V. ZVYAGIN.** Signal-to-noise ratio study of full-field fourier-domain optical coherence tomography. *Appl. Opt.,* 2005, vol. 44, 7722-7729
- **P.D. WOOLIAMS ; R.A. FERGUSON ; C. HART ; A. GRIMWOOD ; P.H. TOMLINS,.** *App. Optics,* 2010, vol. 49 (11), 2014-2021